# EUROPEAN PATENT APPLICATION

(11) **EP 0 550 832 A1**
(43) Date of publication of application: **14.07.1993**
(21) Application number: 92120848.4
(22) Date of filing: 07.12.1992
(51) Int. Cl.: G06F 15/40

(54) **Apparatus for estimating properties of molecules and method therefor**

(30) Priority: 06.01.1992 JP 301/92
(71) Applicant: HITACHI, LTD., Chiyoda-ku, Tokyo 101 (JP)
(72) Inventor: Izawa, Masaru, Hitachi Daiyon, Kokubunji-shi (JP); Ushio, Jiro, Hitachi Daiyon, Kokubunji-shi (JP); Shuji, Toshinobu, Hironocho, Uji-shi (JP)
(74) Representative: Altenburg, Udo, Dipl.-Phys.

(57) **Abstract**

According to the present invention, relations between respective units, each of which comprises an atom chain of π conjugated system or an atomic species, and polarizabilities of the respective units are obtained previously. These are stored in a memory device (32) of a computer system. A test molecule is decomposed to specify units constituting the relevant molecule. The polarizabilities corresponding to the specified respective units are read out from the memory to form the sum total thereof. The sum total thus obtained is a molecular polarizability. As clearly seen from the results indicated in Figs. 8 and 10, the molecular polarizability thus estimated is in good accordance with theoretical and experimental values. Refractive index and dielectric constant can be obtained by using well-known relationships, starting from the polarizability. According to the present invention, it is possible to obtain also an anisotropic polarizability in a similar way as for an isotropic polarizability.

## Description

### BACKGROUND OF THE INVENTION

The present invention relates to an apparatus for estimating properties of molecules and a method therefor and more in detail to an apparatus for estimating molecular polarizability and dielectric constant for electric properties of molecules or refractive index for an optical property and a method therefor.

As a method for estimating theoretically the molecular polarizability there are known the perturbed Hartree - Fock method and the Rayleigh - Schrödinger method which is a method for calculating an electronic state of a molecule. By using these methods it is possible to obtain anisotropic polarizability, taking the orientation of the molecule with respect to electric field (anisotropy) into account. Hereinbelow these methods are called "ab-initio molecular orbital methods".

As a method for obtaining the molecular polarizability on the basis of experimental data there is known a method for obtaining an average value of anisotropic polarizabilities (isotropic polarizability). By this method, starting from experimental data for various sorts of molecules, polarizabilities of different constituent units of a relevant molecule are previously determined by the least squares method, etc. and the polarizability of a test molecule is obtained as a sum of the polarizabilities of the respective units, which have been previously determined. The detail of such a method is described in "Calculation of the Molecular Polarizability Tensor" by K. J. Miller, J. Am. Chem. Soc. (1990) or "A New Empirical Method to Calculate Average Molecular Polarizabilities" by K. J. Miller and J. A. Savchik, J. Am. Chem. Soc. (1979). The documents are herein incorporated by reference.

It is important for elucidating electric properties of molecules and for designing materials to obtain the molecular polarizability with a high precision. It is only the ab-initio molecular orbital method using no experimental data that can theoretically calculate the polarizability with a reliable precision and at present a molecule consisting of about 20 atoms is a limit of molecules, for which the polarizability can be calculated. However materials actually used have great molecular weights. For example, the number of atoms of a polymer is greater than several thousands and therefore it is difficult to calculate theoretically the polarizability with a satisfactory precision. The reason thereof is because both calculation time and memory capacity increase proportionally to the fourth power of the number of atoms and calculation thereof for great molecules having more than 20 atoms exceeds the limit of present computers.

By the method for obtaining the molecular polarizability on the basis of experimental data, since the number of existing experimental data sets was insufficient, although it was possible to obtain the isotropic polarizability, it was difficult to obtain the anisotropic polarizability. This is due to technical difficulties for obtaining the anisotropic polarizability by experiments. Further, by the prior art method bases on experimental data, the polarizability is assigned to several units, each of which is composed of several atoms. For this reason there was a problem that the number of unit data sets was very great. The unit data used here mean the structure of the units and the value of the polarisability thereof. Consequently, in the case where there exist no unit data, the polarizability cannot be calculated. In such a case, a number of experimental values are necessary for adding unit data and it is not easy to add data in reality.

Heretofore there was no efficient method for solving these problems, which was an obstacle for designing a molecule by computer simulation for a molecule having a desired molecular polarizability.

### SUMMARY OF THE INVENTION

A first object of the present invention is to provide an apparatus and a method capable of estimating properties of a molecule having a great number of constituent atoms, i.e. more than 20 atoms.

A second object of the present invention is to provide an apparatus and a method capable of obtaining properties of a molecule in a very short time.

A third object of the present invention is to provide an apparatus and a method capable of obtaining properties of any kind of molecules.

A fourth object of the present invention is to provide an apparatus and a method capable of estimating properties, taking anisotropy of a molecule into account.

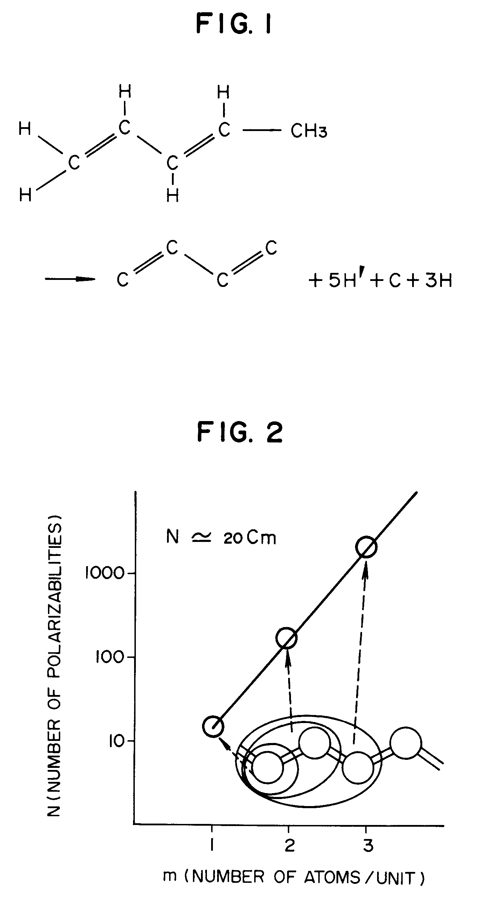

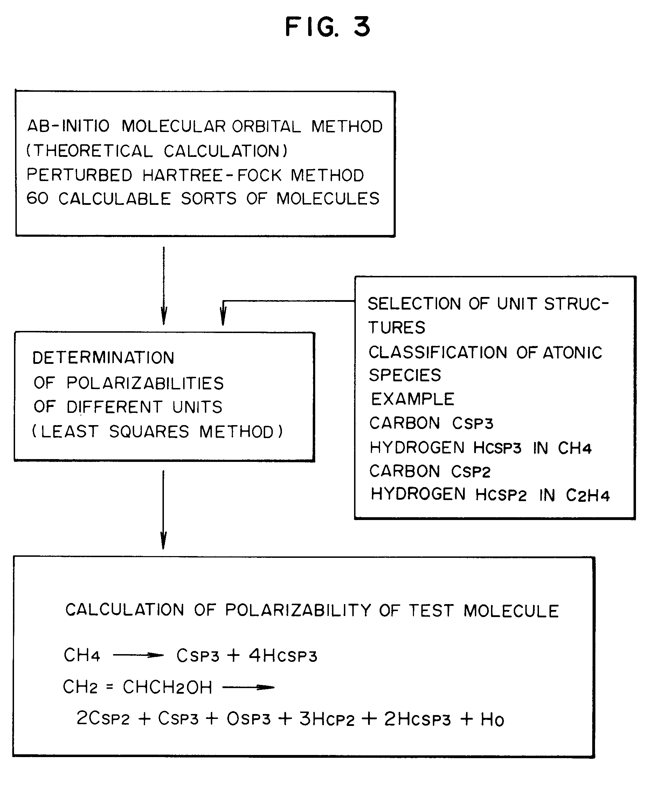

In order to achieve at least one of the above objects, according to the present invention new units are used. An atomic species or an atom chain of π conjugated system corresponds to each of the units. Here the atomic species means atoms classified according to the bonding state thereof. An atom chain of π conjugated system means an atom chain, in which multiple bondings and simple bondings appear alternately. For example, referring to Fig. 1, 1, 3 pentadiene includes units consisting of an atom chain of π conjugated system (C = C - C = C), hydrogen atoms (H') bonded to this atom chain, carbon atoms (C) outside of the conjugate system, and hydrogen atoms (H) bonded to those carbon atoms and the total number of the units is 10.

The molecular polarizability (isotropic) is obtained for each of the units on the basis of the ab-initio molecular orbital method. The ab-initio molecular orbital method is described in Chemical Physics Letters, Vol. 53, No. 3 (1978), pp. 568 - 570 and Chemical Physics Letters, Vol. 102, No. 6 (1983), pp. 544 - 549, which are incorporated herein by reference.

Since it is known that the molecular polarizability is given by a sum of polarizabilities of respective units constituting the molecule (addition law) (refer to Miller and Miller et al. cited previously), according to the present invention the sum total of the polarizabilities of the respective units is calculated to obtain the polarizability of the molecule. Influences of electric field are neglected in the molecular polarizability thus obtained. That is, it is isotropic.

For obtaining the anisotropic molecular polarizability, anisotropic polarizabilities should be previously obtained for the respective units. Factually, they are the polarizability measured in the X direction when electric field in the X direction is applied thereto, that measured in the Y direction when electric field in the Y direction is applied thereto, that measured in the Z direction when electric field in the Z direction is applied thereto, that measured in the Y direction when electric field in the X direction is applied thereto, that measured in the Z direction when electric field in the Y direction is applied thereto, and that measured in the X direction when electric field in the Z direction is applied thereto. The average of these polarizabilities is equal to the isotropic polarizability. The anisotropic polarizability of the molecule can be estimated by adding anisotropic polarizabilities in identical directions to each other.

The present invention is based on a new idea that the molecular species is selected as the unit. When the molecular species is selected as the unit, since an atom is in one unit, the number of unit data sets is not so great and thus addition of data can be easily carried out by calculating small molecules. Fig. 2 is a graph, in which the number of atoms in one unit and the number of polarizabilities per unit are plotted, when a molecule consisting of 20 atoms is assumed. As clearly seen, it can be understood that when the number of atoms per unit increases, the number of polarizabilities, which should be determined for each of the units, increases extremely, which is not realistic.

However this classification gives no satisfactory result for all kinds of molecules and in particular there is a problem for a molecule including a π conjugated system. For such a molecule a π conjugated system is considered to be a unit.

In the present invention, since the isotropic molecular polarizability is equal to the sum of the isotropic polarizabilities of the respective units (atomic species), it is possible to calculate the polarizability of a macromolecule consisting of several ten thousand atoms in a reasonable time with a satisfactory precision. Further it is possible also to calculate the anisotropic polarizability, for which the direction of the molecule is taken into account, by using polarizability data represented by directional components (x, y, z) of the polarizabilities of the respective units (atomic species). By the ab-initio molecular orbital calculation, calculation of the anisotropic polarizability can be effected and preparing data base on the basis thereof, it is possible to obtain data of the anisotropic polarizabilities for the respective units. In this way, it was made possible to obtain easily the anisotropic molecular polarizability, further anisotropic dielectric constant and refractive index, which had been experimentally difficult.

### BRIEF DESCRIPTION OF THE DRAWINGS

These and other objects and technical advantages of the present invention will be readily apparent from the following description of the preferred exemplary embodiments of the invention in conjunction with the accompanying drawings, of which
Fig. 1 is a diagram indicating the relation between a test molecule and units constituting it;
Fig. 2 is a graph indicating the relation between the number of atoms in a unit and the number of polarizabilities necessary for each unit;
Fig. 3 is a flow chart indicating a method for estimating the molecular polarizability in an embodiment;
Fig. 4 is a schematic diagram indicating the construction of an apparatus according to the embodiment;
Fig. 5 is a flow chart indicating the operation of the apparatus according to the embodiment;
Fig. 6 is an example of a list of data which should be inputted in order to obtain an anisotropic polarizability;
Fig. 7 is a diagram indicating an example of display of a result;
Fig. 8 is a graph for comparing results of an embodiment with results obtained by the CPHF method for the polarizability for a plurality of sorts of molecules;
Fig. 9 is a list of molecules, for which the isotropic polarizability has been obtained experimentally;
Fig. 10 is a diagram for comparing results obtained in the embodiment with corresponding experimental values for the molecules indicated in Fig. 9; and
Fig. 11 is a diagram for comparing refractive indices obtained in an embodiment with corresponding experimental values for Nylon 6.

### DESCRIPTION OF THE PREFERRED EMBODIMENTS

Hereinbelow an embodiment of the present invention will be explained in detail.

The method for estimating the molecular polarizability according to the present embodiment will be briefly explained. The polarizability of each of the units and the molecular polarizability are determined by the method described below. At first, the electronic state and the molecular structure of the molecule are obtained by the Hartree - Fock (HF) method and then polarizabilities of several sorts (60 sorts) of small molecules (having numbers of atoms smaller than 20) are calculated on the basis of data thus obtained and by means of the Coupled Perturbed HF (CPHF) method. MIDI-4 is used as the basic function for this calculation and further a polarizability function is added thereto in order to improve calculation precision. The polarizability of each of the units (unit structures) is determined by using the least squares method on the basis of the polarizabilities of these small molecules. The respective units are so selected that the electronic state can be taken into account and that the number of polarizabilities is small. Then, using the obtained polarizabilities of the respective units, the polarizability of the test molecule is calculated as a sum total of the polarizabilities of the corresponding units. For a molecule including a π conjugated system, an atom chain of the π conjugated system is considered as a unit.

Although the CPHF method is used here for determination of the polarizabilities of the respective units, it is also possible to obtain the polarizabilities of the respective units by using the perturbation method or a more precise configuration interaction method to obtain the molecular polarizability on the basis thereof.

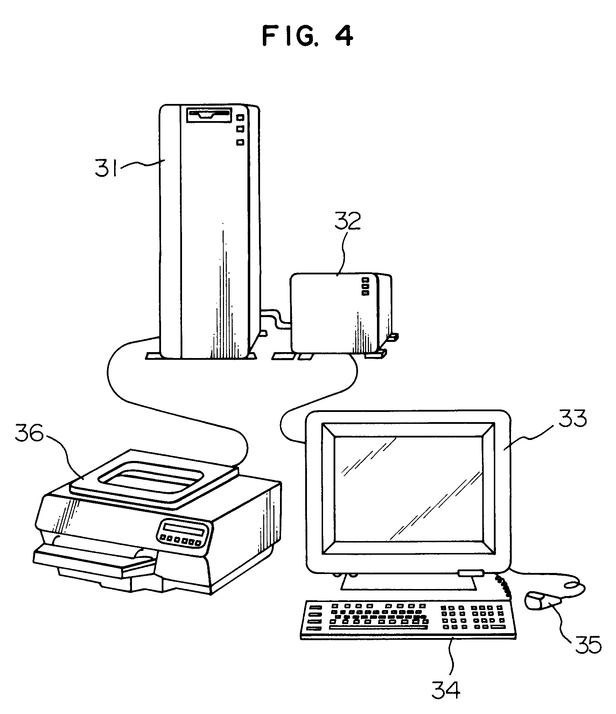

In the present embodiment, the polarizabilities of the respective units described above are incorporated in a computer system, which is constructed as an apparatus for estimating the molecular polarizability. Fig. 4 indicates an example of the computer system for estimating the polarizability, in which polarizability parameters described above are incorporated as a data base. The hardware portion of this apparatus consists of an operation processing device 31 controlling operation control, a disk device 32 storing data, a graphic display 33, a keyboard 34, a mouse 35 and a printer 36. The keyboard 34 is used for inputting data necessary for obtaining refractive index and dielectric constant (according to circumstances) and the mouse 35 is used for effecting operations such as selection of options, translation and rotation of the molecule, and change of the structure. Even if there is no mouse 35, similar operation may be effected only by means of the keyboard 34. The display 33 displays the structure and selected options. Further the polarizabilities of the respective units are arranged in the form of a data base and stored in the disk device 32.

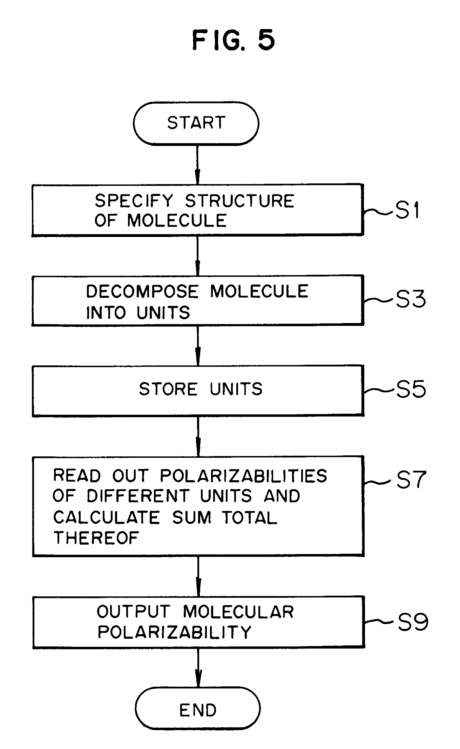

Next, taking a case where the isotropic polarizability of a methane (CH₄) molecule as an example, the operation of this system will be explained. Fig. 5 is a flow chart for explaining the operation.

In STEP 1, when a formula or a name of a molecule is inputted through the keyboard 34, the operation processing device 31 specifies a molecular structure, using existing software. Then the molecule is decomposed so as to specify units constituting the molecule (STEP 3). Concretely speaking, one carbon atom C and 4 hydrogen atoms H constitute respective units. The operation processing device 31 stores the respective units in its register (STEP 5). Next the device 31 determines the polarizabilities of the respective units and stores them in another area of the register by referring to the data base of polarizabilities stored in the disk device 32 (STEP 7). The polarizabilities of the respective units are sent from the register to an adding circuit to obtain the sum total thereof, which is the polarizability (isotropic) of the methane molecule. In STEP 9, the molecular polarizability obtained in STEP 7 is displayed on the display 33 and at the same time it is printed by driving the printer 36.

The example described above is an example for obtaining the isotropic polarizability. Further also for determination of the anisotropic polarizability data were formed, taking the direction of the molecule with respect to the electric field into account in the step for preparing the data base of the polarizabilities of the respective units and it was verified that the polarizability can be determined with a precision almost identical to that obtained for the isotropic polarizability.

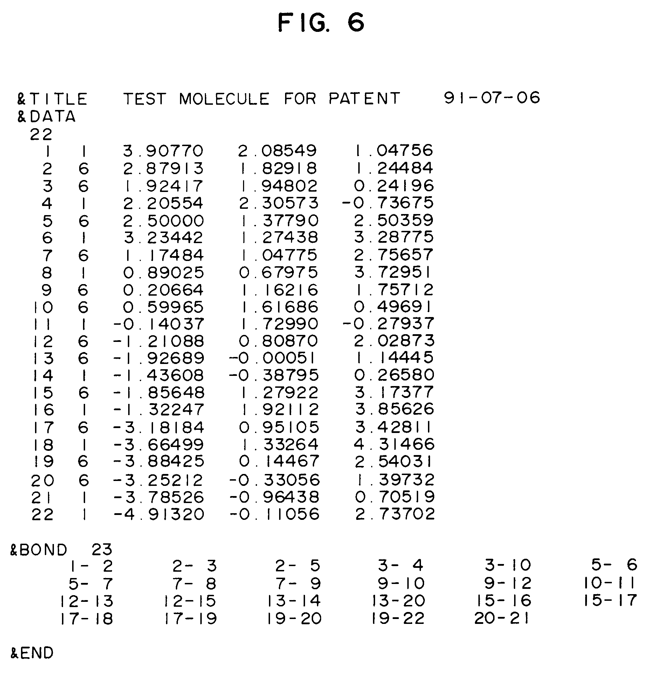

Fig. 6 indicates inputted data for a hydrocarbon molecule having a number of atoms of 22, when the anisotropy is taken into account. It is composed of three-dimensional coordinates of the atoms in the molecule (numbers of six figures), information on the bonding, atomic numbers (1 or 6), title, etc. These data can be obtained from the name of the molecule, using existing soft ware. When such data are inputted, a three-dimensional molecular structure is specified. When such a molecule is decomposed into units, parameters indicating directions of the respective units with respect to a reference point are obtained at the same time. Then the anisotropic polarizabilities of the respective units read out from the data base are corrected by using parameters relating to the directions. The anisotropic polarizability of the molecule can be obtained by adding the polarizabilities thus corrected.

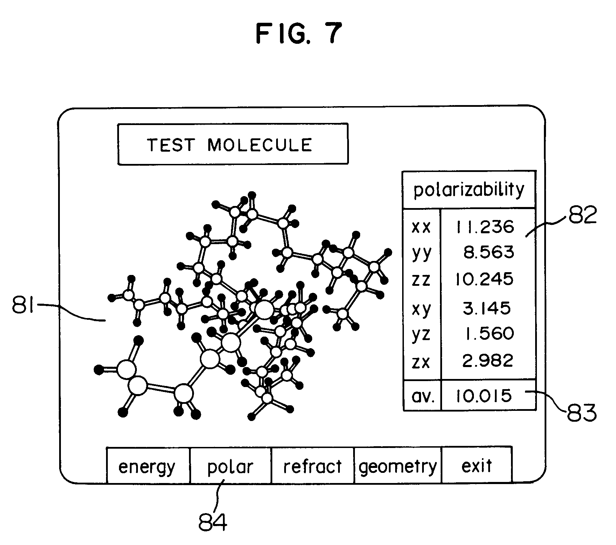

Fig. 7 shows an example of display of the three-dimensional structure 81 of a certain molecule and polarizabilities thereof (anisotropic 82, isotropic 83). The polarizabilities are displayed, when "polar" among selection items 84 on the display is selected by means of the mouse or the function key, etc. (or inputted by means of the keyboard according to circumstances). Further either one of them or only those relating to a certain direction (x, y, z) can be displayed by using an option appearing when "polar" is selected. Still further, for the anisotropic polarizability, when the molecular structure is varied, the operation processing device 31 executes the processing indicated in Fig. 5 with a high speeds In this way the value of the polarizability is recalculated in real time and displayed. Furthermore, as optional functions, a function, by which the units used for estimating the polarizability are displayed with different colors, and a function, by which contributions of the units to the total polarizability are displayed with different colors, are added. In addition, a function, by which values of refractive index and dielectric constant are displayed, when "refract" among the selection items 84 displayed on it is possible to obtain data of the anisotropic polarizabilities for the respective units. In this way, it was made possible to obtain easily the anisotropic molecular polarizability, further anisotropic dielectric constant and refractive index, which had been experimentally difficult the display is selected by means of the mouse or the function key, is added.

Hereinbelow study results on the precision of the molecular polarizability obtained in the above described embodiments will be explained.

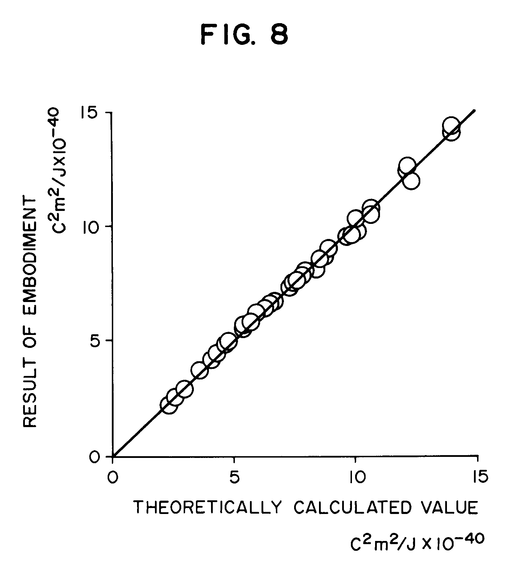

Fig. 8 is a graph, in which molecular polarizabilities obtained in the embodiments (ordinate) are compared with those obtained by the CPHF method (abscissa). The difference between them is about 0.7 % and thus it was verified that they are substantially in accordance with each other.

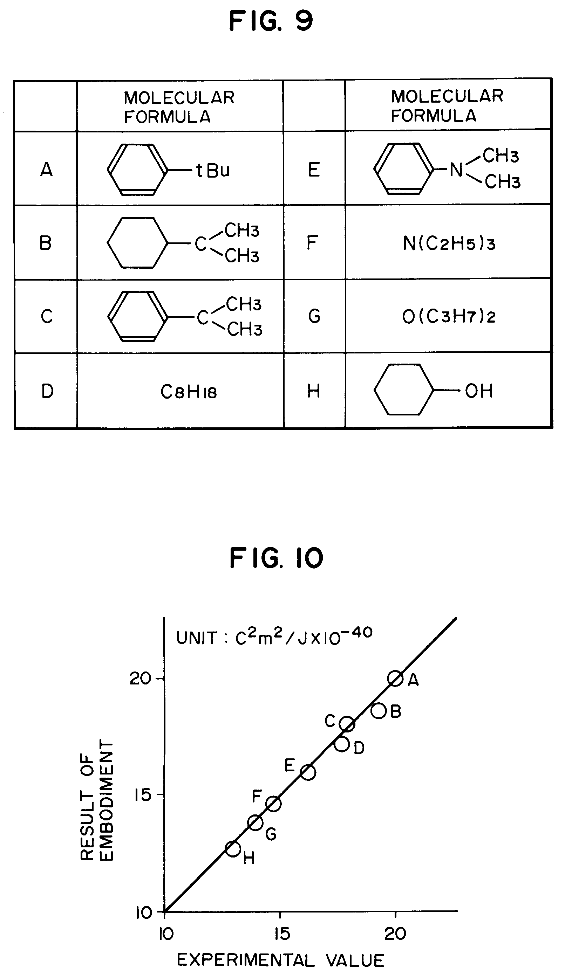

The polarizability (isotropic) of t-butylbenzene (refer to Molecule A in Fig. 9) was calculated by using a large scale computer (fabricated by Hitachi Co. Ltd., trade name M-680H) to execute the CPHF method. This calculation took about 24 hours. On the contrary, if the data base on the polarizabilities of the units is stored, execution of this embodiment takes almost no time.

Next results of comparison between values obtained in this embodiment and experimental values will be explained. The embodiment was executed on molecules as large as possible, for which there exist experimental values. Fig. 9 indicates molecular formulas of the molecules used for this comparison. Taking experimental values into account, calculation values obtained by the molecular orbital method underestimate the polarizability with an almost constant ratio. In order to conform the calculation values to those obtained experimentally, the values obtained in this embodiment were multiplied by 1.14. Adjustment of calculation values by using a coefficient, as described above, is effected also for calculation of molecular vibrational frequency and it has been verified that the coefficient approaches 1.0, if the number of basic functions in the molecular orbital calculation is increased. The graph in Fig. 10 indicates results thus obtained. For a series of these molecules, results of this embodiment (ordinate) reproduce well experimental values (abscissa) and it was found that mean errors is 1.6 %. The alphabetical letters in Fig. 10 correspond to those used in Fig. 9.

The refractive index of a molecule is obtained by putting the molecular polarizability and the molecular density in the Lorenz equation. In a similar way, non-linear optical characteristics of a molecule can be obtained from the molecular polarizability, using the Lalama and Gerito equation.

Dielectric constant can be obtained from the Clausis - Mosotti equation by putting the molecular polarizability in it or the Debye's equation in which the glass transition temperature and the dipole moment are took account.

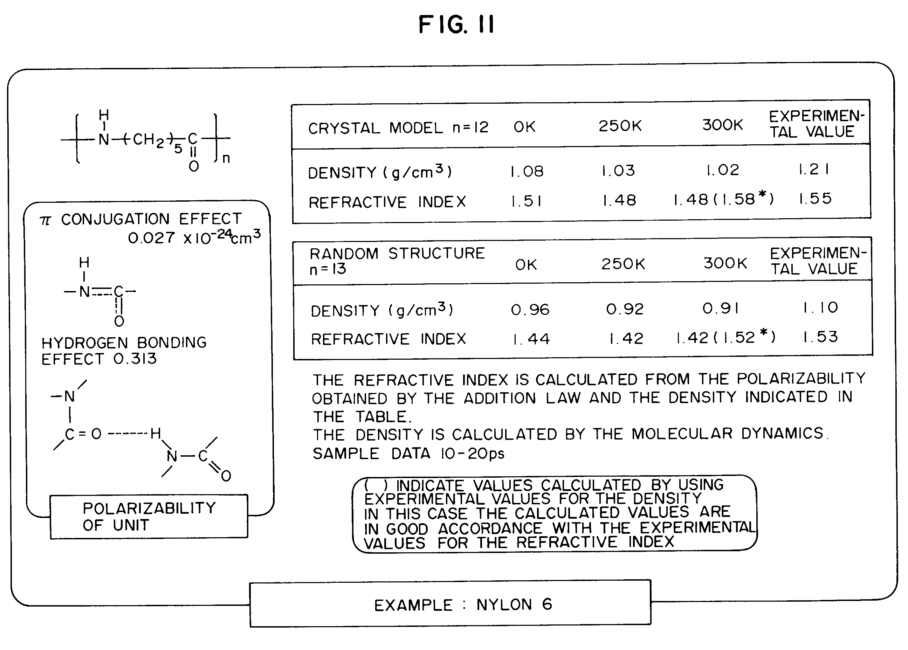

Fig. 11 indicates refractive indices for Nylon 6 obtained on the basis of the molecular polarizability obtained by the method used in the embodiment as well as results of comparison with corresponding experimental values. It can be seen that they are in good accordance with each other. In order to improve further the precision, it is preferable to effect corrections, using the glass transition temperature.

The equations described above and necessary parameters are stored in the disk device 32. The execution of the equations is carried out by the operation processing device 31.

Although, in the above description, organic compounds are dealt with, the method according to the present invention can be applied in a similar way to inorganic compounds. For example, the molecular polarizability of disilane can be calculated as a sum of polarizabilities of two silicon atoms and six hydrogen atoms.

Further polarizability parameters according to the present invention (molecular species and polarizability of the units of the π conjugated system) are used as interatomic potential in molecular dynamics.

As described above, according to the present invention, since
(1) a molecular polarizability of a macro-molecule such as a polymer can be estimated easily by a simple computer simulation;
(2) electric characteristics such as dielectric constant, refractive index, etc. of the molecule can be estimated, starting from the molecular polarizability described above; and
(3) indications for material design can be given by estimating electric characteristics of the material,
   a remarkable effect can be obtained that it is possible to carry out molecule and material design efficiently with a more high efficiency by a computer simulation.

A Japanese Patent Application No. Hei 4 - 301 and U.S. Serial No. 07/543217 are incorporated herein by reference.

The present invention has been described in detail, it should be understood that various changes, substitutions and alternations can be made hereto without departing from the spirit and scope of the present invention as defined by the appended claims.

## Claims

1. An apparatus for estimating properties of molecules comprising:
means (32) for storing relations between predetermined units and polarizabilities corresponding to the respective units, each of said units comprising an atom chain of π conjugated system or an atomic species, the atomic species being atoms classified according to a bonding state thereof;
means (31) for reading out respective polarizabilities of the units constituting a test molecule and obtaining polarizability of the test molecule by processing those polarizabilities; and
means (31) for estimating properties of the test molecule, based on the polarizability of said molecule.

2. An apparatus according to Claim 1, wherein said atomic species is atoms classified according to opposite parties, with which they are bonded.

3. An apparatus according to Claim 1, wherein said polarizabilities corresponding to the respective units are isotropic.

4. An apparatus according to Claim 1, wherein said polarizabilities corresponding to the respective units are anisotropic.

5. An apparatus according to Claim 1, wherein said polarizabilities of the respective units are calculated, based on a known theory.

6. An apparatus according to Claim 5, wherein said known theory is Coupled Perturbed Hartree Fock Method.

7. An apparatus according to Claim 1, wherein said means for obtaining the polarizability calculates a sum total of said polarisabilities of the respective units.

8. An apparatus according to Claim 1, wherein said means for estimating properties of the test molecule estimates at least either one of dielectric constant and refractive index as properties of the test molecule.

9. An apparatus for estimating polarizability of a molecule comprising:
means (32) for storing relations between predetermined units and polarizabilities corresponding to the respective units, each of said units comprising an atom chain of π conjugated system or an atomic species, the atomic species being atoms classified according to a bonding state thereof; and
means (31) for reading out respective polarizabilities of the units constituting a test molecule and obtaining polarizability of the test molecule by processing those polarizabilities.

10. An apparatus according to Claim 9, wherein said atomic species is atoms classified according to opposite parties, with which they are bonded.

11. An apparatus according to Claim 9, wherein said polarizabilities corresponding to the respective units are isotropic.

12. An apparatus according to Claim 9, wherein said polarizabilities corresponding to the respective units are anisotropic.

13. An apparatus according to Claim 9, wherein said polarizabilities of the respective units are calculated, based on a known theory.

14. An apparatus according to Claim 9, wherein said known theory is Coupled Perturbed Hartree Fock Method.

15. An apparatus according to Claim 9, wherein said means for obtaining the polarizability calculates a sum total of said polarizabilities of the respective units.

16. An apparatus for estimating properties of a molecule comprising:
means (32) for storing relations between predetermined units and polarizabilities corresponding to the respective units, each of said units comprising an atom chain of π conjugated system or an atomic species, the atomic species being atoms classified according to a bonding state thereof;
means (31) for specifying structure of a test molecule;
means (31) for decomposing said specified molecule and specifying units constituting said molecule;
means (31) for reading out respective polarizabilities of said units constituting said test molecule and obtaining polarizability of said test molecule by processing those polarizabilities, and
means (31) for estimating properties of said test molecule, based on the polarizability of said molecule.

17. An apparatus according to Claim 16, wherein said atomic species is atoms classified according to opposite parties, with which they are bonded.

18. An apparatus according to Claim 17, wherein said means for specifying the units specifies constituent atoms, opposite parties, with which relevant atoms are bonded, and an atom chain of π conjugated system, starting from a structure of said test molecule.

19. An apparatus according to Claim 16, wherein said polarizabilities corresponding to the respective units are isotropic.

20. An apparatus according to Claim 16, wherein said polarizabilities corresponding to the respective units are anisotropic.

21. An apparatus according to Claim 20, wherein said means for specifying the units specifies constituent atoms, opposite parties, with which relevant atoms are bonded, a bonding direction of each of the atoms, and an atom chain of π conjugated system, starting from a structure of said test molecule.

22. An apparatus for estimating polarizability of a molecule comprising:
means (32) for storing relations between predetermined units and polarizabilities corresponding to the respective units, each of said units comprising an atom chain of π conjugated system or an atomic species, the atomic species being atoms classified according to a bonding state thereof;
means (31) for specifying structure of a test molecule;
means (31) for decomposing said specified molecule and specifying units constituting said molecule; and
means (31) for reading out respective polarizabilities of said units constituting said test molecule and obtaining polarizability of said test molecule by processing those polarizabilities.

23. An apparatus according to Claim 22, wherein said atomic species is atoms classified according to opposite parties, with which they are bonded.

24. An apparatus according to Claim 23, wherein said means for specifying the units specifies constituent atoms, opposite parties, with which relevant atoms are bonded, and molecular chains of π conjugated system, starting from a structure of said test molecule.

25. An apparatus according to Claim 22, wherein said polarizabilities corresponding to the respective units are isotropic.

26. An apparatus according to Claim 22, wherein said polarizabilities corresponding to the respective units are anisotropic.

27. An apparatus according to Claim 26, wherein said means for specifying the units specifies constituent atoms, opposite parties, with which relevant atoms are bonded, a bonding direction of each of the atoms, and an atom chain of π conjugated system, starting from a structure of said test molecule.

28. A method for estimating properties of molecules, comprising the steps of:
storing relations between predetermined units and polarizabilities corresponding to the respective units (STEP 5), each of said units comprising an atom chain of π conjugated system or an atomic species, the atomic species being atoms classified according to a bonding state thereof;
reading out respective polarizabilities of the units constituting a test molecule and obtaining polarizability of a test molecule by processing those polarizabilities (STEP 7); and
estimating properties of the test molecule, based on the polarizability of said molecule (STEP 9).

29. A method for estimating polarizability of a molecule, comprising the steps of:
storing relations between predetermined units and polarizabilities corresponding to the respective units (STEP 5), each of said units comprising an atom chain of π conjugated system or an atomic species, the atomic species being atoms classified according to a bonding state thereof; and
reading out respective polarizabilities of the units constituting a test molecule and obtaining polarizability of a test molecule by processing those polarizabilities (STEP 7).

30. A method for estimating properties of a molecule, comprising the steps of:
specifying structure of a test molecule (STEP 1);
decomposing said specified molecule and specifying units constituting said molecule (STEP 3), each of said units comprising an atom chain of π conjugated system or an atomic species, the atomic species being atoms classified according to a bonding state thereof;
reading out respective polarizabilities of said units constituting said test molecule from storing means, in which relations between predetermined units and polarizabilities corresponding to the respective units are previously stored, and obtaining polarizability of said test molecule by processing those polarizabilities (STEP 7); and
estimating properties of said test molecule, based on the polarizability of said molecule (STEP 9).

31. A method for estimating polarizability of a molecule, comprising the steps of:
specifying structure of a test molecule (STEP 1);
decomposing said specified molecule and specifying units constituting said molecule (STEP 3), each of said units comprising an atom chain of π conjugated system or an atomic species, the atomic species being atoms classified according to a bonding state thereof; and
reading out respective polarizabilities of said units constituting said test molecule from storing means, in which relations between predetermined units and polarizabilities corresponding to the respective units are previously stored, and obtaining polarizability of said test molecule by processing those polarizabilities (STEP 7).
